# EUROPEAN PATENT APPLICATION

(11) **EP 0 645 458 A1**
(43) Date of publication of application: **29.03.1995**
(21) Application number: 93830389.8
(22) Date of filing: 22.09.1993
(51) Int. Cl.: C12P 41/00, C12P 7/40, C07D 295/02, C07C 57/30, C07C 57/34, A01N 43/84

(54) **Chemoenzymatic process for production of S fenpropimorph**

(71) Applicant: CAFFARO S.p.A. Società per l'Industria Chimica ed Elettrochimica, I-20145 Milan (IT)
(72) Inventor: Sunjic, Vitomir, 41001 Zagreb (KR); Gelo, Mirjana, 41001 Zagreb (KR)
(74) Representative: Forattini, Amelia

(57) **Abstract**

A process for stereoselective (enantioselective) production of S-(-)-fenpropimorph, which process comprises reacting 4-*tert*-butylbenzylchloride or bromide with methyldiethylmalonate, decarbethoxylation of the product into racemic 3-(4-*tert*-butylphenyl)-2-methylpropionic acid ethyl ester in DMSO in the presence of alkali cations, then *Pseudomonas sp.* lipase catalyzed resolution of racemic ester to S-(-)-acid, base-catalyzed racemisation and recycling of the R-(+)-ester, acylation of cis-2,6-dimethylmorpholine with the activated derivative of S-(-)-acid, and final reduction of the intermediary amide to provide optically (enantiomerically) pure S-(-)-fenpropimorph. The key enzymatic reaction is fast, employs inexpensive commercially available enzyme, requires minimal work-up, and can therefore easily be performed on a large scale.

## Description

### Description of the Invention

This invention relates to the new method for production of the optically pure (enantiomerically pure compound, EPC) form of S-(-)-1-[3-(4-*tert*-butylphenyl)-2-methylpropyl-*cis*-3,5-dimethylmorpholine (**II**), one of the most important representatives of a new class of systematic fungicides. Racemic form of this compound is commercialized as fungicide under the name fenpropimorph.

It is already known that racemic compounds general formulae **I** possess high and specific fungicidal activity (Ger. Pat. 2,656,747, Ger. Pat. 2,752,135, both to BASF, AG, Ludwigshafen; W. Himmele et al., *Angew. chem. Int. Ed. Engl.*, **19** (1980), 184). They show fungicidal activity against wheat mildew, barley mildew and brown rust of wheat, as confirmed by numerous field trials in control of cereal diseases (K. Bohnen et al., *Proc. Brit. Crop Prot. Conf.-Pest. Dis. **1979***, 541; J.C. Atkin et al., *Proc. Brit. Crop Prot. Conf.-Pest. Dis.* **1981**, 307). Fenpropimorph strongly inhibits Δ-14 reduction in the ergosterol synthesis pathway. When the mildew-susceptible barley varieties were treated with racemic fenpropimorph, strong fungicidal activity was documented (J.K.M. Brown et al., *Crop Prot.* **11** (1992) 44; J.F. Jenkin et al., J*. Agric. Sci.*, **117** (1991) 287). High sensitivity of powder mildew (*Erysiphe graminis*) to fenpropimorph was reported, though some difference was related to geographic regions (F.G. Felsenstein, *Antifungal Compd.*, **1**(1990) 161).

The data presented in literature suggest that the binding conformation of fenpropimorph and other tertiary amine fungicides to Δ-8,14-sterol reductase in fungi correlates with a *pseudo*-1,3-diequatorial orientation of the amine moiety (G.S. Besarab et al., *ACS Symp. Ser. 504* (Synth. Chem. Agrochem III), **1992**, 414, *Chem. Abstr.* **118** (1993) 2375e). The potent *in vitro* inhibition of the plant Δ-5,7-sterol Δ-7-reductase by fenpropimorph was demonstrated (M. Taton et al., *Biochem. Biophys. Res. Commun.*, **181** (1991) 465). In the presence of fenpropimorph sensitive strains of fusarium species accumulated mainly Δ-8,14-sterols, whereas the tolerant ones accumulated either both Δ-8,14 and Δ-8-sterols or only Δ-8-sterols. This indicated that fenpropimorph toxicity is related to sterol Δ-14-reductase sensitivity (D Debieu et al., *Phytochemistry*, **31** (1992) 1223). 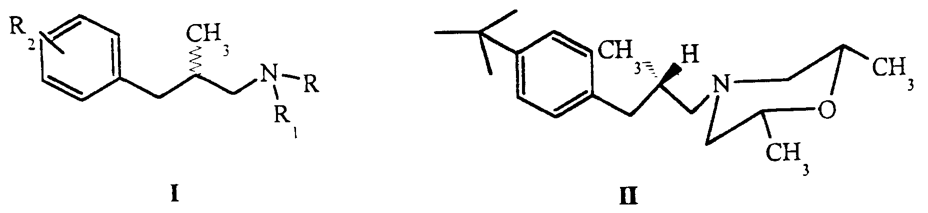
It was demonstrated that the fungicidal activity of 3-phenylpropylamines is also substantially influenced by the conformation of the whole molecule, and configuration of the chiral centre present in 1-(subst.) phenyl-2-methylpropylamino derivatives I (W. Himmele et al., Angew. chem. Int. Ed. Engl., 19 (1980), 184, E. H. Pommer, Pestic. Sci., 15 (1984), 284). From these studies resulted S-(-)-fenpropimorph (II) as the most selective and the specifically active systematic fungicide, which absolute configuration was determined by X-ray structure analysis (W. Himmele et al., Angew. chem. Int. Ed. Engl., 19 (1980), 184), and also its much higher fungicidal activity as compared to its R-(+)-enantiomer, was demonstrated, based on the tests on wheat mildew and brown rust of wheat (Ger Offen. 2,907,614, to Bayer AG).

Random synthesis of a large variety of different amine compounds, as well as intelligent structural modification of the lead structure fenpropimorph, have not lead to a new market product so far. Well over 15000 amines are reported to have been screened at BASF (A. Akers et al., *Pestic. Sci.*, **31**(1991) 521). Rational drug design of azasterol mimics has led to a number of very potent inhibitors of the sterol Δ-1-reductase which also displayed high fungicidal activity in the greenhouse. Although many of these compounds are more powerful reductase inhibitors than racemic fenpropimorph, under field conditions none showed significant advantages over this established fungicide. Most likely, fenpropimorph already exhibits the maximal fungicidal activity which can be attained by blocking sterol Δ-14-reductase. This would mean that, with the development of the second generation amine fungicide racemic fenpropimorph, this class of compounds has already virtually been optimized (A. Akers, et al., *Pestic. Sci.*, **31**(1991) 521; D. Bellus et *al.*, *Angew. Chem. Int. Ed. Engl.*, **30** (1991) 1193).

All above data reveal the importance of our invention of stereoselective production of S-(-)-fenpropimorph, the active enantiomer in the racemic mixture which is presently commercialized. Moreover, elimination of the inactive enantiomer from the commercial product is beneficial both from the economic and ecological point of view, according to the generally accepted trend in production and commercialization of all biologically active compounds (J. Crosby, *Tetrahedron*, **47** (1991) 4789; S.C. Stinson, *Chem. & Eng. News*, **1992**, September 28., 46-79).

Non-stereoselective preparation of S-(-)-fenpropimorph is previously disclosed, leading to the racemic compound **II** and using camphorsulphonic acid as the chiral resolving agent (Ger Offen. 2,907,614, Bayer AG). This method is economically inconvenient, requires chemical manipulation of racemic material, and the half of the final product should be descharged since its recycling *via* racemization is not possible.

We have now found an advantageous chemoenzymatic process for production of S-(-)-**II**, starting from easily available achiral starting materials, as are 4-*tert*-butylbenzylchloride or bromide and methyl-diethylmalonate. In the key step enzymatic kinetic resolution of racemic 3(4-*tert*-butylphenyl)-2-methylpropionic acid ethylester with lipase from *Pseudomonas sp*. is undertaken. This step affords optically pure (+)-3-(4-*tert*-butylphenyl)-2-methylpropionic acid and optically pure (-)-3-(4-*tert*-butylphenyl)-2-methylpropionic acid ethylester. The former possesses the required S-configuration, as suggested the correlation of its [α]_{D} rotation value (+19.9, c=1 in CHCl₃) with that for S-(+)-3-phenyl-2-methyl propionic acid ([α]_{D} +25.6, c=1, in CHCl₃; D.L. Delnick et al, *Tetrahedron Lett*. **47** (1990) 6797), and confirmed by its transformation in S-(-)-fenpropimorph, whereas the ester with "wrong" configuration can easily be racemized and recycled by the enzymatic resolution step. Enantioselective hydrolysis is biocatalyzed by the crude or purified lipase preparations from *Pseudomonas sp.*, which are commercially available, stable, and which can be used on the large biotechnology scale.

Preparation of bromomethylated derivatives of aromatic compounds can be performed either by bromination of the methylaromatic compounds, or by bromomethylation of aromatic compounds. The former method is inconvenient because of the need of expensive reagents like N-bromosuccinimide, and the catalyst of radicalic reactions. Besides, this method ends usually up with an intractable mixture of mono- and dibromo derivatives (see e.g. F. Voegtle et al., *Chem. Ber*. **1973**, 106, 717)

Bromomethylation, however, proceeds slowly in aqueous solutions, otherwise it required use of gaseous hydrogen bromide, and has the disadvantage that often highly toxic bromomethyl methyl esters are formed (see e.g. A. Warshawsky et al., *Polym*. J. **1984**, 16, 234 and cited references). All these inconveniences are recently reported to be eliminated by the usage of solid paraformaldehyde and 30% hydrobromic acid in acetic acid (A.W. van der Made et al., J. Org. *Chem*. **1993**, 58, 1262). The authors noticed highly selective monobromination of methylated benzenes at temperatures below 80 °C, and polybromomethylation at higher temperatures.

Particularly the purposes of the invention can be achieved by a method of production of S-(-)-1-[3-(4-*tert*-butylphenyl)-2-methylpropyl-*cis*-3,5-dimethylmorpholine, comprising the following steps:
(i) the alkylation of a compound of structure (1) 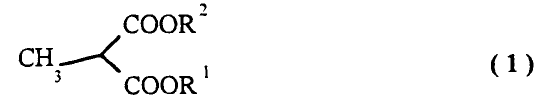 in which R¹ is methyl, ethyl or propyl, R² is methyl, ethyl or propyl, by a compound of structure (2) 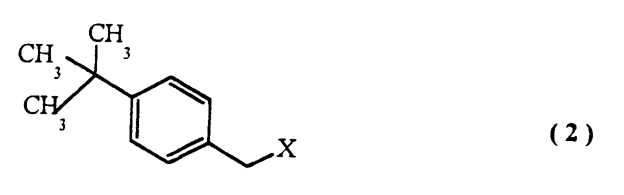 in which X is Cl or Br, to obtain a compound of structure (3) 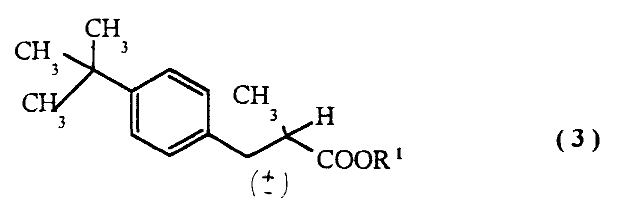 in which R¹ has the same meaning,
(ii) enzymatic, kinetic resolution of said compound of structure (3) using a lipase, to obtain a compound of structure (4) 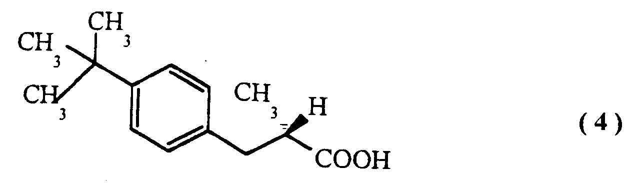 which is S-(+)-3-(4-*tert*-butylphenyl)-2-methylpropionic acid,
(iii) activating said compound of structure (4) to acylate *2,6*-*cis*-dimethylmorpholine to obtain a compound of formula (5) 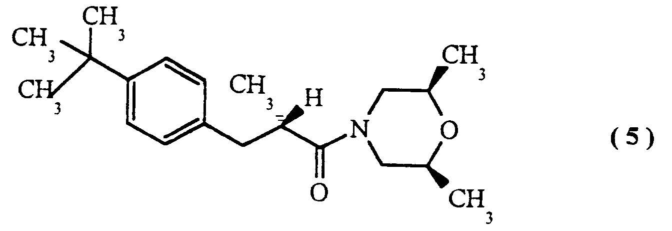 which is S-(-)-3-(4-*tert*-butylphenyl)-2-methylpropionic acid *cis*-dimethylmorpholide,
(iv) reducing said compound of structure (5) to obtain the final, optically pure, product.

Preferably the step (i) comprises the obtainment of an intermediate compound of structure (6) 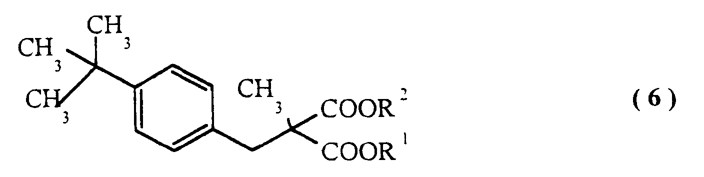
in which R¹ and R² have the same meaning, said compound of structure (6) being decarbethoxylated to obtain said compound of structure (3). According to this preferred embodiment a substantial increase of the yield can be achieved.
Preferably the step (ii) is carried out at a temperature between 25 °C and 50, preferably between 30 °C and 40 °C, and is carried out in an aqueous buffer suspension, preferably a phosphate buffer suspension. A solvent selected in the group of dichloromethane, toluene, light petroleum, cycloexane, and diisopropylether, acetone can be used, particularly acetone is preferred. Preferably the pH is in the range of from about 7 to about 7.5. A compound of structure (7) 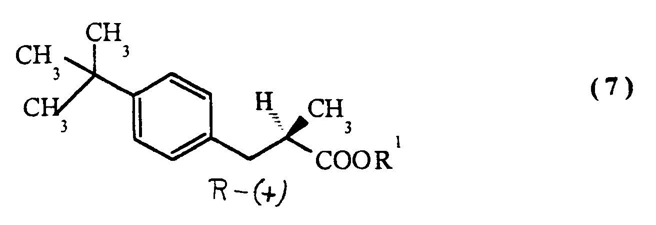
in which R¹ has the same meaning, can be separated from said compound of structure (4). This separation can be carried out by extraction of said compound of structure (7) from the acqueous medium, preferably with hexane or light petroleum, at a pH in the range of about 8 to about 10. Preferably the compound of formula (4) is subsequently extracted by means of a solvent selected from the group of chloroform, dichloromethane and ethylacetate, at a pH in the range of about 0.5 to about 2. According to a different embodiment the separation of the compound of structure (7) can be carried out by chromatography, preferably on silica gel using a solvent selected in the group of: light petroleum chloroform, dichloromethane and dichloroethane.
Once the compound of structure (7) is separated, it can be racemized and recycled at the beginning of said step (ii). The racemization can be carried out by heating at a temperature in the range of 25°C to reflux temperature, preferably at a temperature in the range of 40°C and reflux temperature in a non acqueous solution in the presence of a base.
Preferably the said step (i) is carried out in the presence of a compound selected in the group of phosphoric acid, dimethylsolphoxide (DMSO), examethyl-phosphor-triamide (HMPA) preferably at a temperature in the range of from about 120 to about 180 °C, more preferably in the range of about 150 to about 180 °C.
Preferably the activation of said compound of structure (4) is performed by chloruration with thyonylchloride or triphosgene, preferably in the presence of a catalitic amount of dimethylformamide (DMF).
Preferably the step (iv) is carried out with the use of a complex hydride in aprotic solvents. The complex hydride can be selected in the group of: lithium aluminium hydride, diethoxyethyl lithium aluminium hydride. The aprotic solvent can be selected in the group of ether, tetrahydrofurane and dioxane. Step (iv) can also be carried out by electrochemical reduction under strongly acidic, non-hydrolytic conditions
According to a preferred embodiment of the invention the compound of structure (2) in which X is Br can be obtained by bromomethylation of *tert*-butyl-benzene preferably with paraformaldehyde and hydrobromic acid, more preferably hydrobromic acid is soluted in acetic acid. This embodiment of the invention is particularly advantageous because it allows to start with *tert*-butyl-benzene, which is a compound industrially easily available at low cost and so the method according to the invention is particularly surprising and important.
Surprisingly, we have found that *tert*-butylbenzene reacts very sluggishly with paraformaldehyde/hydrobromic acid in acetic acid at temperatures below 100°C. At higher temperatures and particularly at about 105°C however, rather fast and completely selective monobromomethylation in para position takes place. Even more surprisingly, no acid catalyzed shift of *tert*-butyl group was noticed, and 4-*tert* butylbromomethyl benzene can be isolated in very high yield by simple work-up procedure.
The invention relates also to the new intermediates prepared according to the above described method.
Generally the above "chirotechnological" method for the production of enantiomerically pure S-(-) fenpropimorph eliminates the ecological problem of chemical waste, since it allows recycling of the "wrong" R-enantiomer of the intermediary ester.Our invention can be illustrated by the sequence of steps that represent new, chemoenzymatic approach to the enantiomerically pure S-(-)-II, as outlined in the Scheme 1. 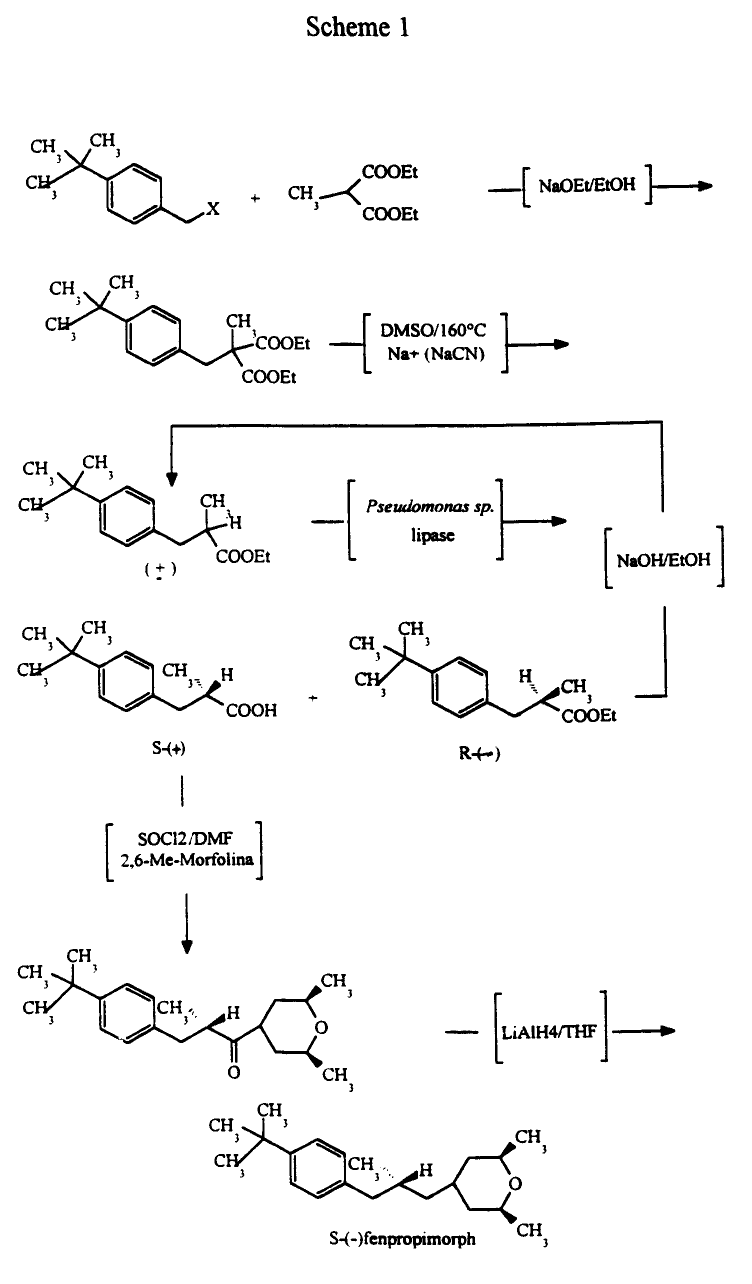
Combining the first two steps into "one-pot" procedure is also possible, as shown in the Examples.
This invention is characterized by the short and chemically simple approach to racemic 3-(4-*tert*-butylphenyl)-2-methylpropionic acid ethylester. Starting from the easily available and inexpensive achiral synthons. The first two steps can also be performed as the "one-pot" process, advantageously using the inorganic salt formed in the first step, as the promotor of thermal decarboxylation in the second step.
This invention is also characterized by complete (nearly 100 % ) enantioselectivity in enzymatic hydrolysis of racemic ester to the desired S-enantiomer of the acid, and by its simple, two step conversion to S-(-)-fenpropimorph. It is also characterized by easy separation of the hydrolyzed, enantiomerically pure acid from the enantiomerically pure ester by selective extraction with some specific solvents. Finally, this invention is characterized by simple recycling of the ester with "wrong" configuration by racemisation in the basic medium, and its subsequent enzymatic resolution.
The invention is illustrated but not limited by the following Examples:

### Example 1.

### 2-(4-tert-Butylbenzyl)-2-methyl-diethylmalonate

To the solution of sodium ethoxide in ethanol, prepared from 1.08 g (47 mmol) of sodium and 60 ml of *abs*. ethanol, 2-methyl-diethylmalonate 7.60 g, 44 mmol), dissolved in 10 ml of *abs*. ethanol, were added dropwise, then the reaction mixture was stirred for 1.5 hr at ambient temperature. To the resulting solution 4-*tert*-buthylbenzyl bromide (9.40 g, 41 mmol), dissolved in 10 ml of abs. ethanol was added. The reaction mixture was stirred for 4 hr at ambient temperature. Then solvent was evaporated, water (80 ml) was added, and crude product (11 g, 84% ) was isolated by extraction (3x50 ml of ether). Pure product was obtained on distillation, b.p. 132132°C/0.02 mm Hg. IR (KBr): 2970, 1750-1730, 1515, 1465, 1380, 1370, 1300, 1280, 1240, 1185, 1105, 1020, 865, 850, 840 cm⁻¹. ¹H-NMR (CDCl₃) δ in ppm; 1.24 (t, 6H), 1.29 (s, 9H), 1.34 (s, 3H), 3.20 (ts 2H), 4.19 (q, 4H), 6.08 (dd, 4H). ¹³C-NMR (CDCl₃) δ in ppm; 13.88, 19.58, 31.19, 34.23, 40.41, 54.67, 61.11, 124.90, 129.70, 132.93, 149.44, 171.90.
Anal., calc'd. for C₁₉H₂₈O₄ (320.43), C 71.22, H, 8.81%. Found; C 71.40, H, 8.95%.

### Example 2.

### rac.-3-(4-tert-Butylphenyl)-2-methylpropionic acid ethylester

Diethylester, obtained as in the Example 1., (1.78 g,, 5.6 mmol), was added to the suspension of sodium cyanide (547 mg, 11.2 mmol) in DMSO (20 ml). The reaction mixture was stirred at ca.160-170°C for 4.5 hr, then water (150 ml) was added and crude product extracted with 3x150 ml of ether. On drying (Na₂SO₄) and evaporation 1.17 g (85%) of crude product were obtained, and purified by distillation; b.p. 85-87°C/0.04 mm Hg. IR (KBr): 2970, 1740, 1515, 1465, 1380, 1370, 1160, 1185, 1125, 1110, 1050, 1020, 865, 840, 815 cm⁻¹. ¹H-NMR (CDCl₃) δ in ppm, 1.14 (d, 3H), 1.17 (t, 3H), 1.30 (s, 9H), 2.60-2.74 (s, 1H), 2.98 (q, 2H, benzylic CH₂), 4.08 (q, 2H, ethylic CH₂), 7.18 (dd, 4H). ¹³C-NMR (CDCl₃) δ in ppm; 13.88, 16.54, 31.15, 34.09, 39.00, 41.20, 59.88, 124.89, 128.39, 136.06, 148.70, 175.79.
Anal., calc'd. for C₁₆H₂₄O₂ (248.34), C 77.38, H, 9.74%. Found; C 77.21, H, 9.96%.

### Example 3.

### rac.3-(4-tert-Butylphenyl)-2-methylpropionic acid

Diethyl ester prepared in the Example 1. (504 mg, 2.0 mmol), was added to a solution of KOH (1.00 g, 18.0 mmol) in 17 ml of abs EtOH, and stirred at ambient temperature for 5 hr. Crude product was obtained on evaporation of the solvent, addition of water (20 ml), adjusting pH to 2, and extraction with ether (3x20 ml); yield 766 mg (86%). Crystallization from ethanol-water afforded pure product, m.p. 107-109°C. IR (KBr): 2960, 1715, 1520, 1460, 1420, 1365, 1290, 1270, 1240, 1195, 1105, 940, 815 cm⁻¹. ¹H-NMR (CDCl₃) δ in ppm; 1.18 (d, 3H), 1.30 (s, 9H), 2.70 (m, 1H), 3.08 (d, 2H), 7.21 (dd, 4H). ¹³C-NMR (CDCl₃) δ in ppm; 14.68, 31.38, 34.37, 41.20, 125.29, 128.67, 135.95, 149.21, 182.73. Anal., calc'd. for C₁₄H₂₀O₂ (220.31), C 76.33, H, 9.15%. Found; C 76.59, H, 8.89%.

### Example 4.

### (+)-3-(4-tert-Butylphenyl)-2-methylpropionic acid

*Comment:* The method described here is the result of the screening of the lipases from the following microbiological sources: *Candida cylindracea*, *Pseudomonas fluorescens*, *Penicillium camemberti*, *Aspergillus niger*, *Geotrichium candidum*, *Candida lipolytica*, *Humicola lanuginosa*, and *Pseudomonas sp*.
*General Procedure.-To* 320 ml of K-phosphate buffer, pH 7.2, 100 mg of *Pseudomonas* *sp.* lipase (Amano, extracellular enzyme of non-specified activity) was added first, then a solution of 1.00 g of racemic ester obtained as in the Example 2, in 35 ml of acetone. The reaction was followed by GLC using HP-17 (crosslinked 50% Ph-Me silicone) column, at injector temp. 220°C, column temperature 155°C, and detector temp. 250°C, Rt of ester 9.27 min, Rt of acid 11.62 min. After 24 hr at 30°C the conversion was nearly 50%. Alter adjusting pH to 1, and extraction with ethylacetate, crude ester and acid are separated on silicagel column using chloroform as eluant. Optical purity of (+)-acid was determined by repeated crystallization from ethanol-water, m.p. 101-103°C; optically pure sample has [α]_{D} +19.9 (c=2.1, in.CHCl₃).
Anal., calc'd. for C₁₄H₂₀O₂ (220.31), C 76.33, H, 9.15%. Found; C 76.14, H, 9.03%.
Optical purity of the remaining ester was determined by ¹H-NMR, on addition of the chiral shift reagent (+)-Eu(hfc)₃, and was found to be >99%.

### Example 5.

### (S)-(-)-N-[3-(4-tert-butylphenyl)-2-methyl-propionyl]-cis-2,6,-dimethylmorpholine

Starting from 290 mg (1.22 mmol) of (+)-acid, its chloride is prepared by heating in thionylchloride (5.0 ml), in the presence of 3-4 drops of DMF. On evaporation of the excess of the reagent, crude acid chloride was used for acylation of cis-2,6-dimethylmorpholine (170 mg, 1.5 mmol). Acylation was performed in dry pyridine at ambient temperature. Solvent was evaporated azeotropically on addition of toluene, crude product was dissolved in dichloromethane, organic extract was washed with dill. (1:4) aqueous HCI, dried (Na₂SO₄) and evaporated. Crude product (320 mg, 83%) was purified by chromatography on silicagel column with chloroform light petroleum (8:2) as eluant [α]_{D} + 25.4° (c=1, in CHCl₃). ¹H-NMR (CDCl₃) δ in ppm; 1.7-2.2 (m, 3H), 2.29 (s, 9H), 3.1-4.5 (m ,7H), 7.1-7.3 (dd, 4H). ¹³C-NMR (CDCl₃) δ in ppm; 18.45, 31.15, 37.19, 42.07, 46.84, 51.02, 70.76, 71.04, 125.38, 128.50, 136.62, 148.09, 174.44.
Anal., calc'd. for C₂₀H₃₁NO₂ (317.47), C 75.67, H, 9.84, N 4.41%. Found; C75.57, H, 9.85, N 4.48%.

### Example 6.

### (S)-(-)-N-[3-(4-tert-butylphenyl)-2-methylpropyl]-cis-2,6,-dimethylmorpholine

Starting from the S-(-)-amide (1.5 g, 5.0 mmol), prepared as in the Example 5, dissolved in THF (20 ml), reduction was performed with LiAlH₄ (2.0 g) dissolved in 8 ml of THF. Reduction was performed at 50°C, during 8 hrs. Crude product was isolated after dropwise addition of 1.2 ml of water, filtration of the inorganic precipitate, evaporatin of the solvent addition of water (20 ml), adjusting pH to 6.5, and distillation of the product (1.18 g, 78%), b.p. 160-170°C/0.8-.1.0 mm Hg, [α]_{D} -3.13° (c=1, in CHCl₃).

### Example 7.

### rac.-3-(4-tert-Butylphenyl)-2-methylpropionic acid ethylester

Starting from 4-tert-butylbenzyl chloride (10.0 g, 55 mmol) and methyl-diethylmalonate (10.5 g, 60 mmol), alkylation was performed in EtONa/EtOH as described in the Example 1. Ethanolic solution of the crude product was adjusted to pH ca. 7 by addition of dill. solution of HCl/EtOH. Solvent was evaporated in *vacuo*, then 150 ml of DMSO were added and residual ethanol distilled off until internal temperature rised to 140°C. The reaction was continued under stirring for 8 hr; crude final product was isolated as described in the Example 2. It was obtained 10.7 g (78%) of the racemic 3-(4-*tert*-butylphenyl)-2-methylpropionic acid ethylester.

### Example 8

### (+)-3-(4-tert-Burylphenyl)-2-methylpropionic acid

*Pseudomonas sp*. lipase (1.0 g) was slurried in 500 ml of K-phopsphate buffer, pH 7.2, and then a solution of 2.0 g of racemic 3-(4-*tert*-butylphenyl)-2-methylpropionic acid ethylester was added dissolved in 50 ml of acetone. After *ca*. 10 hrs at 30°C 50% (GLC) of the ester was hydrolized, and then pH was adjusted to 9.0. The resulting slurry was extracted with 200 ml of hexane, organic extracts were collected, dried (Na₂SO₄), and evaporated to afford 1.05 g of crude (+)-ester. Aqueous phase was adjusted to pH 1.0 with aq. HCl (1:1), and extracted with chloroform (3x200 ml). Organic extracts were dried (Na₂SO₄), evaporated, and crude (-)-acid was crystallized from ethanol-water, 0.76 g, [α]_{D} +20.0 (c=2, in.CHCl₃).

### Example 9.

### Racemization of R-(+)-3-(4-tert-butylphenyl)-2-methylpropionic acid ethyl ester

The unreacted (+)-ester, as obtained in the Example 4 (1.0 g), was dissolved in abs. ethanol (15 ml), to which a few milligrams of sodium were previously added. On brief heating under reflux, H₂O the base-catalyzed racemization is completed. Then pH was adjusted to 6.57, and solvent evaporated *in vacuo*. Crude product can be slurried in phosphate buffer and recycled by the enzymatic kinetic resolution without ulterior purification.

### Example 10

### 4-tert-Butylbenzylbromide

To the slurry of formaldehyde (1.85 g, 60 mmol), and *tert*-butylbenzene (6.7 g, 55 mmol, Aldrich), in glac. acetic acid (25 ml), 33% HBr in acetic acid (10 ml) was added under stirring at 40100°C. Soon alter, clear pale-yellow solution formed, and stirring was continued at 105 °C for 6 hrs. Reaction solution was poured on crushed ice (200 g), and extracted with light petroleum (3x80 ml). Combined organic extracts were dried, evaporated, and crude, chromatographically pure product (11.35 g, 87%) distilled, b.p. 140-145 °C /68 mmHg. ¹H-NMR (CDCl₃) δ in ppm; 1.29 (s, 9H), 4.45 (s, 2H), 7.32 (4H). 13C-NMR (CDCl₃) δ in ppm; 31.95, 33.35, 125.56, 128.61, 134.49, 151.35.

## Claims

1. Method of production of S-(-)-1-[3-(4-*tert*-butylphenyl)-2-methylpropyl-*cis*-3,5-dimethylmorpholine, comprising the following steps:
(i) the alkylation of a compound of structure (1) 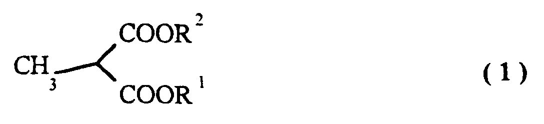 in which R¹ is methyl, ethyl or propyl, R² is methyl, ethyl or propyl, by a compound of structure (2) 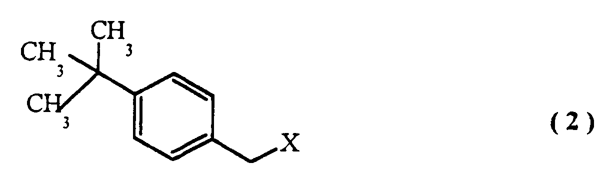 in which X is Cl or Br, to obtain a compound of structure (3) 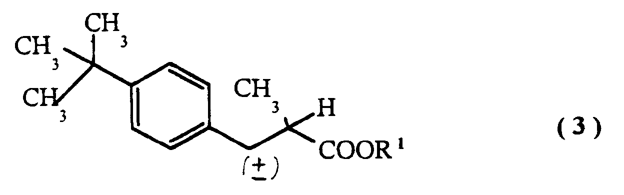 (+) in which R¹ has the same meaning,
(ii) enzymatic, kinetic resolution of said compound of structure (3) using a lipase, to obtain a compound of structure (4) 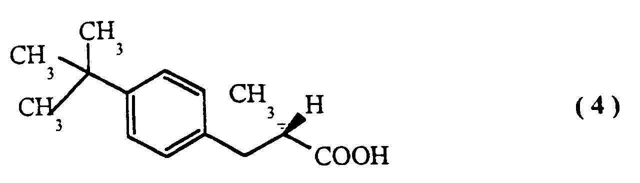 which is S-(+)-3-(4-*tert*-butylphenyl)-2-methylpropionic acid
(iii) activating said compound of structure (4) to acylate 2,6-*cis*-dimethylmorpholine to obtain a compound formula (5) 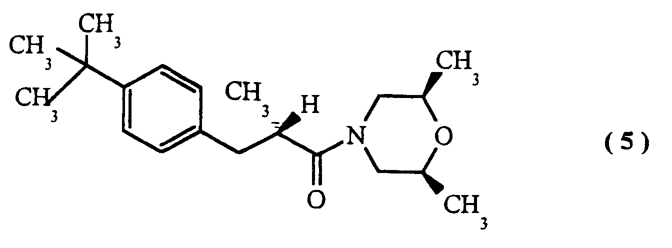 which is S-(+)-3-(4-*tert*-butylphenyl)-2-methylpropionic acid *cis*-dimethylmorpholide,
(iv) reducing said compound of structure (5) to obtain the final, optically pure, product.

2. Method according to claim 1 in which said step (i) comprises the obtainment of an intermediate compound of structure (6) 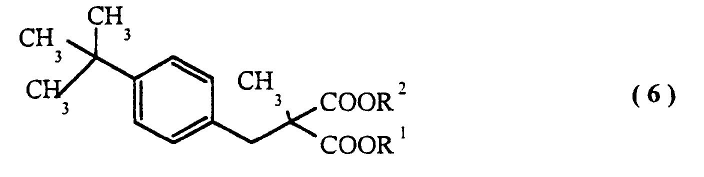 in which R¹ and R² have the same meaning, said compound of structure (6) being decarbethoxylated to obtain said compound of structure (3).

3. Method according to at least one of the preceding claims in which said lipase is obtainable from *Pseudomonas sp*.

4. Method according to at least one of the preceding claims in which said step (ii) is carried out at a temperature between 25 °C and 50, preferably between 30 °C and 40 °C.

5. Method according to at least one of the preceding claims in which said step (ii) is carried out in an acqueous buffer suspension, preferably a phosphate buffer suspension.

6. Method according to at least one of the preceding claims in which said step (ii) is carried out in the presence of a solvent selected in the group of dichloromethane, toluene, light petroleum, cycloexane, and diisopropylether, acetone.

7. Method according to at least one of the preceding claims in which said step (ii) is carried out at a pH in the range of from about 7 to about 7.5

8. Method according to at least one of the preceding claims in which said step (ii) comprises a separation of a compound of structure (7) 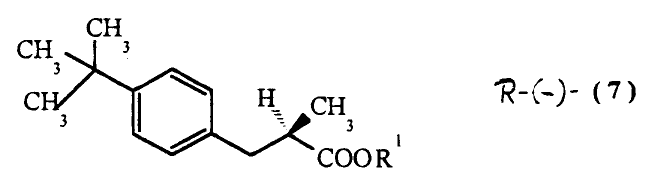 in which R¹ has the same meaning, from said compound of structure (4).

9. Method according to claim 8 in which said separation is carried out by extraction of said compound of structure (7) from the acqueous medium, preferably with hexane or light petroleum, at a pH in the range of about 8 to about 10.

10. Method according to claim 9 comprising the subsequent extraction of said compound of formula (4) by means of a solvent selected from the group of chloroform, dichloromethane and ethylacetate, at a pH in the range of about 0.5 to about 2.

11. Method according to claim 8 in which said separation is carried out by chromatography, preferably on silica gel using a solvent selected in the group of: light petroleumchloroform, dichlorom-thane and dichloroethane.

12. Method according to at least one of claims 8 to 11 in which said compound of structure (7) is racemized and recycled at the beginning of said step (ii).

13. Method according to claim 11 in which said racemization is carried out by heating at a temperature in the range of 25°C to reflux temperature, preferably at a temperature in the range of 40°C and reflux temperature in a non acqueous solution in the presence of a base.

14. Method according to at least one of the preceding claims in which said step (i) is carried out in the presence of a compound selected in the group of phosphoric acid, dimethylsolphoxide (DMSO), hexamethyl-phosphortriamide (HMPA) preferably at a temperature in the range of from about 120 to about 180 °C, more preferably in the range of about 150 to about 180°C.

15. Method according to at least one of the preceding claims in which said activation of said compound of structure (4) is performed by chloruration with thyonylchloride or triphosgene, preferably in the presence of a catalitic amount of dimethylformamide (DMF).

16. Method according to at least one of the preceding claims in which said step (iv) is carried out with the use of a complex hydride in aprotic solvents.

17. Method according to claim 16 in which said complex hydride is selected in the group of: lithium aluminium hydride, diethoxyethyl lithium aluminium hydride.

18. Method according to claim 16 or 17 in which said aprotic solvent is selected in the group of ether, tetrahydrofurane and dioxane.

19. Method according to at least one of the preceding claims in which said compound of structure (2) in which X is Br is obtained by bromomethylation of *tert*-butylbenzene preferably with paraformaldehyde and hydrobromic acid, more preferably hydrobromic acid is soluted in acetic acid and the reaction is carried out at a temperature higher than 100 °C.

20. A compound of structure (4) 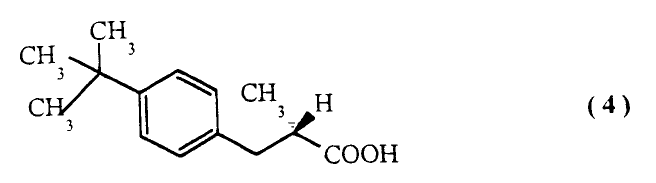 which is 3-(4-*tert*-butylphenyl)-2-methylpropionic acid.

21. A compound according to claim 20 in enantiomerically pure S-(+) form

22. A compound of structure (5) 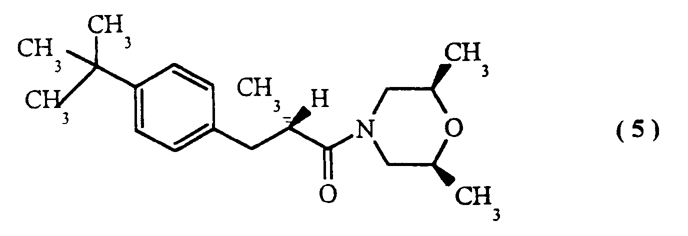 which is S-3-(4-*tert*-butylphenyl)-2-methylpropionic acid *cis*-dimethylmorpholide.

23. A compound according to claim 22 in enantiomerically pure S-(+) form.

24. A compound of structure (6) 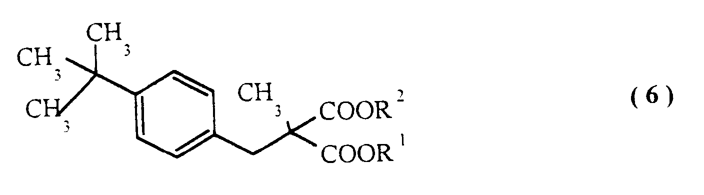 in which R¹ is methyl, ethyl or propyl, R² is methyl, ethyl or propyl,.

25. A compound of structure (3) 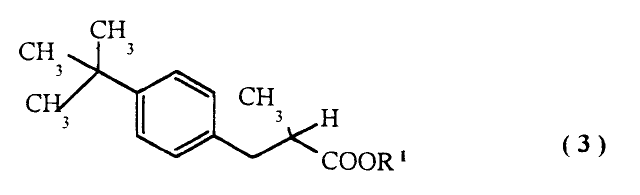 in which R1 is methyl, ethyl or propyl.
